# EUROPEAN PATENT APPLICATION

(11) **EP 3 545 950 A1**
(43) Date of publication of application: **02.10.2019**
(21) Application number: 18461541.7
(22) Date of filing: 26.03.2018
(51) Int. Cl.: A61K 9/16, A61K 31/496, A61K 9/20

(54) **PHARMACEUTICAL COMPOSITION COMPRISING BREXPIPRAZOLE**

(71) Applicant: Adamed sp. z o.o., 05-152 Czosnów k/Warszawy (PL)
(72) Inventor: GARBERA, Kamil, 59-220 Legnica (PL); WOS-LATOSI, Katarzyna, 05-092 Lomianki (PL)
(74) Representative: Sulikowski, Daniel

(57) **Abstract**

The present invention relates to a pharmaceutical composition comprising a granulate comprising brexpiprazole, wherein the granulate is obtained by wet-granulation of a carrier using a granulation liquid that is a solution of brexpiprazole in a solvent system. The present invention relates also to a granulate used in the pharmaceutical composition, and a unit dosage form comprising the pharmaceutical composition, and the method for manufacturing a pharmaceutical composition comprising brexpiprazole.

## Description

This invention relates to a pharmaceutical composition comprising brexpiprazole, and a unit dosage form comprising the pharmaceutical composition. The present invention also relates to a specific granulate in the pharmaceutical composition.

Brexpiprazole (7-[4-(4-benzo[b]thiophen-4-yl-piperazin-1-yl)butoxy]-1H-quinolin-2-one), sold under the brand name Rexulti(R), is a relatively new D2 dopamine and serotonin 1A partial agonist, described as a serotonin-dopamine activity modulator (SDAM), and a potent antagonist of serotonin 2A receptors, noradrenergic alpha 1B and 2C receptors. Brexpiprazole has been approved for the treatment of schizophrenia, and as an adjunctive treatment for major depressive disorder (MDD). Brexpiprazole was disclosed for the first time in PCT publication WO2006112464, and has the following structure:

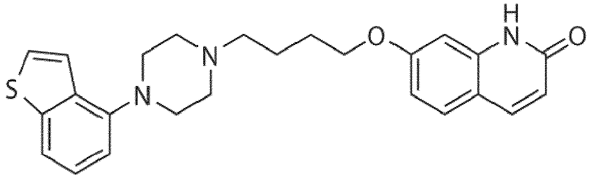

Brexpiprazole has poor solubility in water, therefore to improve its solubility, special measures must be taken. In general, the methods for increasing the solubility of poorly soluble compounds are known in the art. For example, one such method is careful selection of excipients by incorporation of solubilizes, and another involves reducing the poorly soluble compound particle size distribution to a range of several to several dozen micrometers.

Such a reduction needs special equipment, such as mills or spray-dryers. Poorly soluble compounds can undergo degradation under conditions used in the process of particle size distribution reduction, and such a separate process step can also cause weight loss.

Low solubility of brexpiprazole has been approached in several ways.

European patent application EP2767285 (Ostuka) relates to a tablet comprising brexpiprazole as the active ingredient. The tablet also includes lactose, cornstarch, microcrystalline cellulose or like excipient, low-substituted hydroxypropyl cellulose, croscarmellose sodium, sodium carboxymethyl starch or like disintegrant; and hydroxypropyl cellulose or like binder. The tablet has favorable disintegration abilities and produces reasonable results in dissolution tests. The method for producing the tablet comprises granulating mixture comprising brexpiprazole, an excipient (a), a binder (b), and a disintegrant (c), and further mixing thereto a lubricant (d). In this method, brexpiprazole remains as a solid during the granulation process.

In turn, European patent application EP 2797631 (Otsuka) relates to a pharmaceutical composition comprising brexpiprazole and a substituted betacyclodextrin. Beta-cyclodextrines improve the solubility of poorly soluble substances by forming an inclusion complex that shows good water-solubility.

Unfortunately, it is not the case that only the careful selection of excipients, binders, disintegrants and other ingredients customarily used in pharmaceutical compositions is important for the development of a composition with favorable solubility characteristics. The particle size distribution of brexpiprazole also plays a key role: it strongly affects the dissolution profile of pharmaceutical compositions comprising brexpiprazole, and therefore causes problems with the development of a pharmaceutical formulation with a specified dissolution profile.

Therefore, it is desirable to develop a pharmaceutical composition comprising brexpiprazole that provides good solubility and eliminates the influence of brexpiprazole particle size distribution on dissolution.

This goal is achieved by the pharmaceutical composition of the invention.

In the first aspect, the invention relates to a pharmaceutical composition comprising a granulate comprising brexpiprazole, wherein the granulate is obtained by wet-granulation of a carrier using a granulation liquid that is a solution of brexpiprazole in a solvent system.

Such a pharmaceutical composition allows for completely eliminating an influence of brexpiprazole particle size distribution on dissolution of brexpiprazole. Furthermore, surprisingly the dissolution process can be easily controlled by the surface area of the carrier used in the pharmaceutical composition of the invention. The inventor observed that the higher the surface area of the carrier, the faster the dissolution of the brexpiprazole. This observation is another very important feature of the invention.

Brexpiprazole can be present in the pharmaceutical composition in any suitable amount. However, the applicant found that the pharmaceutical composition should comprise 0.20 wt.% - 15 wt.% brexpiprazole. Due to the relatively low dose of brexpiprazole used in the therapy, the amounts of the pharmaceutical composition in the invention comprising more than 15 wt.% brexpiprazole would be too small and inconvenient for daily administration. Amounts lower than 0.20 wt.% would be unsuitable due to the large final dosage form, which could cause swallowing difficulties. Preferably, the pharmaceutical composition comprises 0.25 wt.% - 5 wt.% of brexpiprazole, which fulfills patient compliance aspects.

The wet-granulation used in the granulation step can be any such process. Preferably, however, the wet-granulation is performed using a fluid-bed granulation process. More preferably, top spray fluid-bed granulation process.

Preferably, the solution comprises brexpiprazole, a solvent system, a binder, and a optional surfactant.

The solvent system is a mixture of at least two solvents that provide a complete dissolution of brexpiprazole. Selection of the proper solvent system is a crucial step. The applicant found that the preferred solvent system is a mixture of acetone, ethanol, and water. More preferably, the acetone, ethanol and water are used in a mass ratio 7.7:1:1. This mass ratio of the solvent system allows for dissolving 1g of brexpiprazole in 252g of the solvent system.

The surfactant is a substance that is generally a compound which lowers the surface tension between two liquids or between a liquid and a solid. Preferably, in the invention, the surfactant is selected from polyoxyethylene fatty alcohol ethers, sorbitan fatty acid esters, polyoxyethylene fatty acid esters, sorbitan esters, glycerol monostearate, polyethylene glycols, cetyl alcohol, cetostearyl alcohol, stearyl alcohol, poloxamers, potassium laureate, triethanolamine stearate, sodium lauryl sulfate, alkyl polyoxyethylene sulfates, benzalkonium chloride, cetyltrimethylammonium bromide, or chitosans. More preferably, the surfactant is selected from polysorbate 80, poloxamer 188, sorbitan monooleate, polysorbate 60, or polyethylene glycols.

The binder is any pharmaceutically acceptable compound that has binding properties. Preferably, in the invention, the binder is selected from Arabic gum, methylcellulose, hydroxyethylcellulose, hydroxypropylcellulose, L-hydroxypropylycellulose (low-substituted), hydroxypropyl methylcellulose (HPMC), sodium carboxymethylcellulose, carboxymethylhydroxyethylcellulose, starch, polyvinylpyrrolidone, or polyvinyl caprolactam-polyvinyl acetate- polyethylene glycol copolymer. More preferably, the binder is selected from polyvinyl caprolactam-polyvinyl acetate-polyethylene glycol copolymer, polyvinylpyrrolidone, hydroxypropyl methylcellulose, hydroxypropylcellulose, or Arabic gum.

The carrier in the invention fills out the size of a composition, making it practical to produce and convenient for the consumer to use. The carrier is a base for active ingredients, which are physically connected to the surface of the carrier. Preferably, in the invention, the carrier is selected from calcium carbonate, calcium phosphate, dibasic calcium phosphate, tribasic calcium phosphate, calcium carboxymethylcellulose, cellulose, dextrin and its derivatives, dextrose, fructose, lactitol, lactose, starch and modified starches, magnesium carbonate, magnesium oxide, magnesium aluminum silicate, isomalt, mannitol, maltitol, maltodextrin, maltose, sorbitol, starch, sucrose, and xylitol, erythritol or a mixture thereof, or a pellet. More preferably, the carrier is selected from lactose monohydate, magnesium aluminum silicate, microcrystalline cellulose, isomalt, mannitol, dibasic calcium phosphate, or a mixture thereof, or a pellet. The pellets used, can be selected from microcrystalline cellulose pellets, sugar pellets, lactose pellets or a mixture thereof.

In the second aspect, the invention relates to a granulate comprising brexpiprazole obtained by wet-granulation of a carrier using a granulation liquid that comprises a solution of brexpiprazole in a solvent system.

Preferably, brexpiprazole is present in the granulate in 0.3 wt.% - 20 wt.%. Due to relatively low doses of brexpiprazole used in the therapy, amounts of the pharmaceutical composition comprising more than 20 wt.% brexpiprazole in the final dosage form would be too small for daily administration, and amounts lower than 0.3 wt.% are unsuitable due to the large final dosage form, which could cause swallowing difficulties.

Preferably, the wet-granulation is performed using a fluid-bed granulation process. More preferably, top spray fluid-bed granulation process.

Preferably, the solution of brexpiprazole comprises brexpiprazole, a solvent system, a binder, and an optional surfactant.

Preferably, the solvent system is a mixture of acetone, ethanol and water. More preferably, the acetone, ethanol and water are used in a mass ratio 7.7:1:1.

Preferably, the surfactant is selected from polyoxyethylene fatty alcohol ethers, sorbitan fatty acid esters, polyoxyethylene fatty acid esters, sorbitan estersn, glycerol monostearate, polyethylene glycols, cetyl alcohol, cetostearyl alcohol, stearyl alcohol, poloxamers, potassium laureate, triethanolamine stearate, sodium lauryl sulfate, alkyl polyoxyethylene sulfates, benzalkonium chloride, cetyltrimethylammonium bromide, or chitosans. More preferably, the surfactant is selected from polysorbate 80, poloxamer 188, sorbitan monooleate, polysorbate 60, or polyethylene glycols.

Preferably, the binder is selected from Arabic gum, methylcellulose, hydroxyethylcellulose, hydroxypropylcellulose, L-hydroxypropylycellulose (low-substituted), hydroxypropyl methylcellulose (HPMC), sodium carboxymethylcellulose, carboxymethylene, carboxymethylhydroxyethylcellulose, starch, polyvinylpyrrolidone, or polyvinyl caprolactam-polyvinyl acetate-polyethylene glycol copolymer. More preferably, the binder is selected from polyvinyl caprolactam-polyvinyl acetate-polyethylene glycol copolymer, polyvinylpyrrolidone, hydroxypropyl methylcellulose, hydroxypropylcellulose, or Arabic gum.

Preferably, the carrier is selected from calcium carbonate, calcium phosphate, dibasic calcium phosphate, tribasic calcium sulfate, calcium carboxymethylcellulose, cellulose, dextrin and its derivatives, dextrose, fructose, lactitol, lactose, starch and modified starches, magnesium carbonate, magnesium oxide, magnesium aluminum silicate, isomalt, mannitol, maltitol, maltodextrin, maltose, sorbitol, starch, sucrose, and xylitol, erythritol or a mixture thereof, or a sugar pellet. More preferably, the carrier is selected from lactose monohydate, magnesium aluminum silicate, microcrystalline cellulose, isomalt, mannitol, dibasic calcium phosphate, or a mixture thereof, or a sugar pellet.

In the third aspect, the invention relates to a unit dosage form comprising the pharmaceutical composition of the invention.

The preferred unit dosage form is a tablet or a capsule. Other unit dosage forms have been discussed in previous studies.

The fourth aspect relates to the method for manufacturing a pharmaceutical composition comprising brexpiprazole, with the following steps:
a) Preparation of a solution of binder, brexpiprazole and optional surfactant in a solvent system.
b) Wet-granulation of a carrier with the solution obtained in a).
All preferable binders, solvent systems, carriers, optional surfactants are as described above for the pharmaceutical composition of the invention. Preferably, the wet-granulation method is fluid-bed granulation, and more preferably top spray fluid-bed granulation.

In the fifth aspect, the invention relates to the use of a wet-granulation process with a solution of brexpiprazole as the granulation liquid in preparation of the pharmaceutical composition comprising brexpiprazole. Such an approach allows for completely eliminating the influence of brexpiprazole particle size distribution on the dissolution profile of bexpiprazole. Control of the dissolution process of brexpiprazole can be achieved by varying the amount of carrier.

The pharmaceutical composition of the invention or a unit dosage form prepared using the composition or the granulate of the present invention may be used for any therapeutic treatment of schizophrenia, and as an adjunctive treatment for major depressive disorder (MDD).

While the present invention has been described in terms of its specific embodiments, certain modifications and equivalents will be apparent to those skilled in the art and are intended to be included within the scope of the present invention.

### EXAMPLES

### Example 1. Preparation of the pharmaceutical compositions of the invention.

All examples were prepared in accordance with the following method. Brexpiprazole used in the study has the following characteristics D [v, 0.5] 209.3 D [v, 0.9] 313.4µm.

The proposed manufacturing process comprises the following steps:
1) Preparation of an appropriate solvent mixture and heating it up to 50°C.
2) Adding and dissolving the binder and surfactant/solubilizer, continuously stirring.
3) Adding the API and stirring until the clear, yellowish solution is obtained.
4) Spraying the API solution onto the carrier and drying the material.
5) Adding the extragranular phase ingredients.
6) Preparing the final dosage form by tablet compression or the encapsulation process.

Fluid-bed granulation was utilized as a granulation step.

The crucial step of the manufacturing process is solution preparation. This requires a mixture of organic solvents (acetone, ethanol) and water in an appropriate mass ratio 7,7:1:1. Every single gram of active compound must be dissolved in 252g of a given mixture, however solubility has not been precisely determined and an improvement of API concentration in the solution is still possible.

After API solution preparation, the given mixture is sprayed onto the carrier/diluent particles of the intragranular phase. Preferably, spraying (granulation) is performed by a top spray fluid-bed granulation process. The product temperature during the process should not exceed 35°C. Once the granulation is completed, the material is dried to 2%w/w moisture content.

The last step is tablet compression or encapsulation, preceded by optional addition of the extragranular phase ingredients. Tablet compression or encapsulation can be completed using standard procedures in pharmaceutical formulations.

Examples of compositions are as follows.

| **Example 1** | | | **Strength 0.25mg** | | **Strength 4.0mg** | |
|---|---|---|---|---|---|---|
| **Tablet** | **Ingredient** | **Functionality** | **%** | **mg/UDF** | **%** | **mg/UDF** |
| **IG** | Brexpiprazole | Active substance | 0.25 | 0.25 | 4.00 | 4.0 |
| | Polysorbate 80 | Surfactant | 4.00 | 4.0 | 4.00 | 4.0 |
| | Polyvinyl caprolactam-polyvinyl acetate-polyethylene glycol copolymer | Binder/ solubilizer | 1.20 | 1.2 | 1.20 | 1.2 |
| | Lactose Monohydrate | Carrier/ diluent | 68.55 | 68.6 | 64.80 | 64.8 |
| | Magnesium Aluminum Silicate | Carrier/ diluent | 10.00 | 10.0 | 10.00 | 10.0 |
| **ExG** | Microcrystalline cellulose | Diluent | 10.00 | 10.0 | 10.00 | 10.0 |
| | Crospovidone | Disintegrant | 5.00 | 5.0 | 5.00 | 5.0 |
| | Magnesium Stearate | Lubricant | 1.00 | 1.0 | 1.00 | 1.0 |
| | Total | | **100.0** | **100.0** | **100.0** | **100.0** |

| | | | | | | |
|---|---|---|---|---|---|---|
| IG, intragranular phase ingredients, ExG, extragranular phase ingredients, UDF, unit dosage form. | | | | | | |

| **Example 2 Tablet** | **Ingredient** | **Functionality** | **%** | **mg/UDF** |
|---|---|---|---|---|
| **IG** | Brexpiprazole | Active substance | 4 | 4 |
| | Poloxamer 188 | Solubilizer | 2 | 2 |
| | Povidone K25 | Binder | 5 | 5 |
| | Microcrystalline Cellulose | Carrier | 83 | 83 |
| **ExG** | Crospovidone | Disintegrant | 5 | 5 |
| | Magnesium Stearate | Lubricant | 1 | 1 |
| | Total | | **100** | **100** |

| | | | | |
|---|---|---|---|---|
| IG, intragranular phase ingredients, ExG, extragranular phase ingredients, UDF, unit dosage form. | | | | |

| **Example 3 Tablet** | **Ingredient** | **Functionality** | **%** | **mg/ UDF** |
|---|---|---|---|---|
| **IG** | Brexpiprazole | Active substance | 4 | 4 |
| | Sorbitan monooleate | Solubilizer | 3 | 3 |
| | HPMC | Binder | 6 | 6 |
| | Isomalt | Carrier | 60 | 60 |
| **ExG** | Microcrystalline cellulose | Diluent | 20 | 20 |
| | Sodium starch glycolate | Disintegrant | 5 | 5 |
| | Sodium stearate fumarate | Lubricant | 2 | 2 |
| | Total | | **100** | **100** |

| | | | | |
|---|---|---|---|---|
| IG, intragranular phase ingredients, ExG, extragranular phase ingredients, UDF, unit dosage form. | | | | |

| **Example 4 Tablet** | **Ingredient** | **Functionality** | **%** | **mg/UDF** |
|---|---|---|---|---|
| **IG** | Brexpiprazole | Active substance | 4 | 4 |
| | Polysorbate 60 | Solubilizer | 2 | 2 |
| | HPC | Binder | 3.5 | 3.5 |
| | Mannitol | Carrier | 72.5 | 72.5 |
| **ExG** | Lactose monohydrate | Diluent | 10 | 10 |
| | Croscarmellose sodium | Disintegrant | 6 | 6 |
| | Sodium stearate fumarate | Lubricant | 2 | 2 |
| | Total | | **100** | **100** |

| | | | | |
|---|---|---|---|---|
| IG, intragranular phase ingredients, ExG, extragranular phase ingredients, UDF, unit dosage form. | | | | |

| **Example 5 Tablet** | **Ingredient** | **Functionality** | **%** | **mg/UDF** |
|---|---|---|---|---|
| **IG** | Brexpiprazole | Active substance | 4 | 4 |
| | Poloxamer 188 | Solubilizer | 4 | 4 |
| | Arabic gum | Binder | 4 | 4 |
| | Sugar pellets | Carrier | 64 | 64 |
| **ExG** | Crospovidone | Disintegrant | 3 | 3 |
| | Microcrystalline cellulose | Diluent | 20 | 20 |
| | Magnesium stearate | Lubricant | 1 | 1 |
| | Total | | **100** | **100** |

| | | | | |
|---|---|---|---|---|
| IG, intragranular phase ingredients, ExG, extragranular phase ingredients, UDF, unit dosage form. | | | | |

| **Example 6 Tablet** | **Ingredient** | **Functionality** | **%** | **mg/UDF** |
|---|---|---|---|---|
| **IG** | Brexpiprazole | Active substance | 4 | 4 |
| | Polyethylene glycol | Solubilizer | 3.5 | 3.5 |
| | Arabic gum | Binder | 3.5 | 3.5 |
| | Dibasic calcium phosphate | Carrier | 50 | 50 |
| **ExG** | Crospovidone | Disintegrant | 5 | 5 |
| | Microcrystalline cellulose | Diluent | 32 | 32 |
| | Magnesium stearate | Lubricant | 2 | 2 |
| | total | | **100** | **100** |

| | | | | |
|---|---|---|---|---|
| IG, intragranular phase ingredients, ExG, extragranular phase ingredients, UDF, unit dosage form. | | | | |

| **Example 7 Tablet** | **Ingredient** | **Functionality** | **%** | **mg/UDF** |
|---|---|---|---|---|
| **IG** | Brexpiprazole | Active substance | 4.4 | 4.0 |
| | Polysorbate 80 | Solubilizer | 1.1 | 1.0 |
| | Polyvinyl caprolactam-polyvinyl acetate-polyethylene glycol copolymer | Binder | 4.5 | 4.0 |
| | Magnesium aluminum silicate | Carrier | 60.0 | 54.0 |
| | Crospovidone | Disintegrant | 3.0 | 2.7 |
| **ExG** | Crospovidone | Disintegrant | 3.0 | 2.7 |
| | Microcrystalline cellulose | Diluent | 23.0 | 20.7 |
| | Magnesium stearate | Lubricant | 1.0 | 0.9 |
| | Total | | **100** | **90** |

| | | | | |
|---|---|---|---|---|
| IG, intragranular phase ingredients, ExG, extragranular phase ingredients, UDF, unit dosage form. | | | | |

| **Example 8 Tablet** | **Ingredient** | **Functionality** | **%** | **mg/UDF** |
|---|---|---|---|---|
| IG | Brexpiprazole | Active substance | 4 | 4 |
| | HPC | Binder | 3.5 | 3.5 |
| | Microcrystalline cellulose | Carrier | 74.5 | 74.5 |
| ExG | Lactose monohydrate | Diluent | 10 | 10 |
| | Croscarmellose sodium | Disintegrant | 6 | 6 |
| | Sodium stearate fumarate | Lubricant | 2 | 2 |
| | Total | | **100** | **100** |

| | | | | |
|---|---|---|---|---|
| IG, intragranular phase ingredients, ExG, extragranular phase ingredients, UDF, unit dosage form. | | | | |

| **Example 9 Capsule** | **Ingredient** | **Functionality** | **%** | **mg/UDF** |
|---|---|---|---|---|
| **IG** | Brexpiprazole | Active substance | 4 | 4 |
| | Polysorbate 80 | Solubilizer | 4 | 4 |
| | Povidone | Binder | 4 | 4 |
| | Microcrystalline cellulose pellets | Carrier | 88 | 88 |
| Capsule fill | Total | | **100** | **100** |

| | | | | |
|---|---|---|---|---|
| IG, intragranular phase ingredients, ExG, extragranular phase ingredients, UDF, unit dosage form. | | | | |

### Example 2. Dissolution profiles for Examples 1 and 7

### Dissolution data

Dissolution tests were carried out in acetate buffer pH 4.5, 900mL, paddle apparatus, 50 RPM using Rexulti 4 mg; batch No. BPS00116, in Example 1 and Example 7.

| **Time [min]** | **Rexulti 4 mg; batch No. BPS00116** | | **Example 1 (Strength 4 mg)** | | **Example 7** | |
|---|---|---|---|---|---|---|
| | **Mean** | **RSD** | **Mean** | **RSD** | **Mean** | **RSD** |
| 5 | 56.0 | 9.8 | 65.5 | 4.3 | 80.5 | 2.9 |
| 10 | 74.7 | 6.3 | 86.3 | 1.2 | 88.2 | 2.1 |
| 15 | 84.2 | 5.1 | 93.6 | 1.9 | 92.2 | 2.8 |
| 20 | 89.3 | 3.7 | 95.6 | 1.1 | 96.1 | 2.0 |
| 30 | 92.6 | 3.0 | 99.6 | 1.8 | 97.7 | 3.1 |

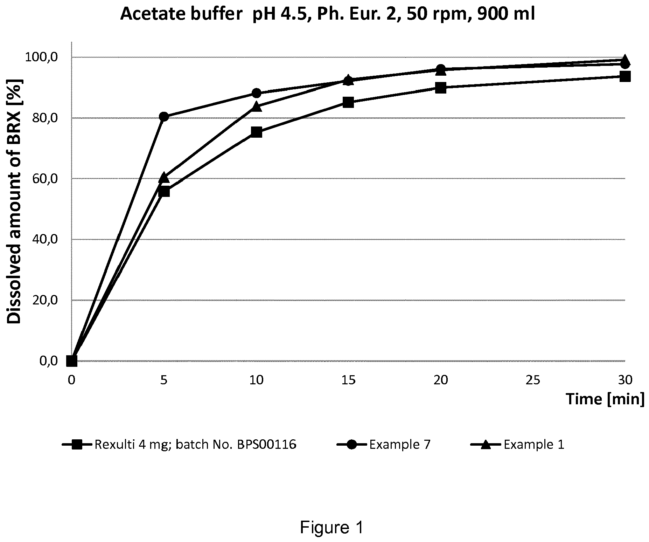

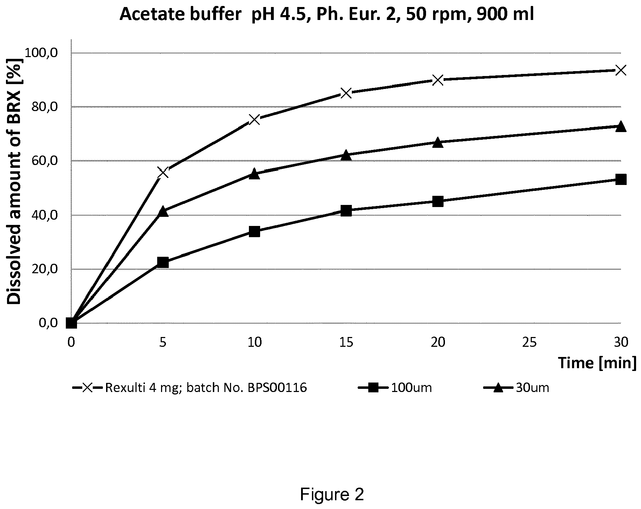

A comparison of the dissolution profiles for Rexulti 4mg BN. BPS00116 and Examples 1 and 7 for 4mg strengths, is presented in Figure 1.

Dissolution results show good a dissolution rate, comparable and even faster than the reference drug product. Example 7 demonstrates faster drug release than Example 1 due to the bigger surface area of the carrier. The carrier used in Example 7 is magnesium aluminum silicate (Neusilin(R)), which has a very large surface area. Example 1 is based on a mixed carrier consisting of magnesium aluminum silicate and lactose monohydrate, in an approximate weight ratio 1:7. Incorporation of such a high amount of lactose monohydrate with a low surface area leads to a lower dissolution profile. Thus, by specific selection of carriers in terms of their surface areas, it is possible to control very precisely the dissolution profiles, and to adjust them if needed.

The presented technology is an interesting improvement to manufacturing processes described in EP 2767285 A1. The big advantage of the pharmaceutical composition of the invention is the ability to eliminate the particle size distribution of brexpiprazole by virtue of dissolving brexpiprazole in an appropriate solvent system, and spraying it onto carrier particles. The brexpiprazole used for the preparation of tablets in Example 1 and Example 7 was of a coarse grade, with particle size distribution as below:

| **PSD** | **D [v,0.1]** | **D [v,0.5]** | **D [v,0.9]** |
|---|---|---|---|
| Brexpiprazole (batch: 85.164.s.3) | 108.8µm | 209.3µm | 313.4µm |

As an additional proof of the pharmaceutical composition of the invention, reference tablets were prepared to demonstrate its performance with different brexpiprazole particle size distribution. Brexpiprazole batch 185.164.s.3 was milled down to obtain two particle size distributions. Sample 1 was characterized by D09 of around 100µm and Sample 2 had D09 of around 30µm. Using these two samples of brexpiprazole, the tablets were prepared according to the technology described in EP 2767285 A1. Dissolution profile results clearly show that reference tables are sensitive to brexpiprazole particle size distribution and their solubility strongly depends on particle size distribution. On the contrary, tablets prepared from the pharmaceutical composition of the invention, with a brexiprazole D09 as high as 300µm, gave a dissolution profile comparable to that of Rexulti.

| Components* | Sample 1 (100µm) | | Sample 2 (30µm) | |
|---|---|---|---|---|
| | % | mg/tab | % | mg/tab |
| Brexpiprazole (D09, 100µm) | 4.45 | 4.0 | - | - |
| Brexpiprazole (D09, 30µm) | - | - | 4.45 | 4.0 |
| Lactose monohydrate | 49.33 | 44.4 | 49.33 | 44.4 |
| Maize starch | 22.22 | 20.0 | 22.22 | 20.0 |
| Microcrystalline cellulose MCC 301 | 11.11 | 10.0 | 11.11 | 10.0 |
| Low-substituted Hydroxypropyl cellulose L-HPC (LH 11) | 11.11 | 10.0 | 11.11 | 10.0 |
| Hydroxypropyl cellulose HPC EF | 1.11 | 1.0 | 1.11 | 1.0 |
| Magnesium stearate | 0.67 | 0.6 | 0.67 | 0.6 |
| *It was assumed that such qualitative and quantitative composition corresponds to that for Rexulti. | | | | |

### Example 3. Stability data

Stability studies were performed for Example 1 tablets (4mg), packed in PVdC blisters. The blisters were placed in stability chambers in stressed conditions (40°C 75%RH). The tablets were analyzed by HPLC after 1, 2 and 3 months and compared to the starting point.

| Results at starting point | Sum of impurities |
|---|---|
| Example 1 | 0.14 |
| Rexulti TM 4mg Tablet (US market, Batch No. BPS00115A) | 0.36* |
| *According to IP.com Number: IPCOM000245389D | |

| | Example 1 | | | |
|---|---|---|---|---|
| | Start | 1 month 40°C 75%RH | 2 month 40°C 75%RH | 3 month 40°C 75%RH |
| Sum of impurities | 0.14 | 0.24 | 0.15 | <LOQ |

Therefore, the pharmaceutical compositions according to the present invention provided not only a superior dissolution rate, comparable to the solutions disclosed in prior studies, but also significant stability even after 3 months in stress conditions.

## Claims

1. A pharmaceutical composition comprising a granulate comprising brexpiprazole, wherein the granulate is obtained by wet-granulation of a carrier using a granulation liquid that is a solution of brexpiprazole in a solvent system.

2. A pharmaceutical composition of claim 1, wherein the pharmaceutical composition comprises 0.20 wt.% - 15 wt.% brexpiprazole.

3. The pharmaceutical composition of any one of the preceding claims, wherein the granulate is obtained using fluid-bed granulation.

4. The pharmaceutical composition of any one of the preceding claims, wherein the solution of brexpiprazole comprises brexpiprazole, a solvent system, a binder, and a optional surfactant.

5. The pharmaceutical composition of claim 4, wherein the solvent system is a mixture of acetone, ethanol, and water.

6. The pharmaceutical composition of claim 5, wherein acetone, ethanol, and water are used in a mass ratio 7.7:1:1.

7. The pharmaceutical composition of claim 4, wherein the surfactant is selected from polysorbate 80, poloxamer 188, sorbitan monooleate, polysorbate 60, or polyethylene glycol.

8. The pharmaceutical composition of claim 4, wherein the binder is selected from polyvinyl caprolactam-polyvinyl acetate-polyethylene glycol copolymer, polyvinylpyrrolidone, hydroxypropyl methylcellulose, hydroxypropylcellulose, or Arabic gum.

9. The pharmaceutical composition of claims 1-8, wherein the carrier is selected from lactose monohydate, magnesium aluminum silicate, microcrystalline cellulose, isomalt, mannitol, dibasic calcium phosphate, or a mixture thereof, or a pellet.

10. Granulate comprising brexpiprazole obtained by wet-granulation of a carrier using a granulation liquid, wherein the granulation liquid comprises a solution of brexpiprazole in a solvent system.

11. The granulate of claim 10, wherein the carrier, solution of brexpiprazole, and solvent system are as defined in claims 1-9.

12. Unit dosage form comprising the pharmaceutical composition as defined in claims 1-9.

13. Method for manufacturing a pharmaceutical composition comprising brexpiprazole with the following steps:
a) Preparation of a solution of binder, brexpiprazole and optional surfactant in a solvent system.
b) Wet-granulation of a carrier with the solution obtained in a).

14. Method of claim 13, wherein the solvent system is a mixture of acetone, ethanol, and water.

15. Method of claims 13 or 14, wherein the carrier, binder and surfactant are as defined in any one of the preceding claims.

16. Method of claims 13-15, wherein the wet-granulation is fluid-bed granulation.

17. Use of a wet-granulation process with a solution of brexpiprazole as the granulation liquid, in preparation of a pharmaceutical composition comprising brexpiprazole.
